# NEW EUROPEAN PATENT SPECIFICATION

(11) **EP 0 472 633 B2**
(45) Date of publication and mention of the opposition decision: **24.11.1999**
(45) Mention of the grant of the patent: 17.02.1993
(21) Application number: 90908711.6
(22) Date of filing: 15.05.1990
(51) Int. Cl.: A61F 13/46

(54) **A DISPOSABLE ABSORBENT ARTICLE WHICH COMPRISES A HOSE-LIKE ABSORPTION BODY**
ABSORBIERENDER WEGWERFARTIKEL MIT EINEM HOSENFÖRMIGEN ABSORPTIONSTEIL
ARTICLE ABSORBANT JETABLE INCORPORANT UN CORPS ABSORBANT EN FORME DE TUBE

(30) Priority: 16.05.1989 SE 8901739
(43) Date of publication of application: 04.03.1992
(73) Proprietor: SCA Hygiene Products AB, 405 03 Göteborg (SE)
(72) Inventor: RUNEMAN, Bo, S-433 75 Partille (SE); RÖNNBERG, Peter, S-431 33 Mölndal (SE)
(74) Representative: Harrison, Michael Charles
(86) International application number: SE9000324
(87) International publication number: WO9014063

(56) References cited:
- EP-A- 0 122 803
- EP-A- 0 173 068
- EP-A- 0 343 941
- EP-B- 0 223 486
- GB-A- 2 124 907
- US-A- 4 795 453

## Description

The present invention relates to a disposable absorbent article, such as a disposable diaper or an incontinence guard, which comprises an inner liquid-permeable casing sheet which when the article is worn, lies nearest the body of the wearer, an outer liquid-impermeable casing sheet or backing sheet, and an absorbent pad which is enclosed between the two casing layers, the absorbent pad includes in at least the region which forms the crotch part of the article when said article is worn a body of shape-stable material and of high liquid permeability, the body being placed centrally in the pad and extending in the longitudinal direction of the article bordering on the inner casing sheet and being surrounded by absorbent material at least on the side thereof remote from said inner casing sheet.

By suitable selection of absorbent material, such as a combination of cellulose-fluff and so-called superabsorbent material, and by appropriate treatment of these materials, such as compression of the fluff material, it is possible to provide such absorbent articles with absorbent pads which possess a satisfactory total absorption capacity, by which is meant the maximum amount of liquid that can be absorbed throughout the whole of the pad. Any leakage experienced when using such products is therefore normally due to the fact that an excessive amount of liquid has been excreted locally onto the pad too rapidly for the capillaries in the fluff material to be able to transport the liquid to drier parts of the absorption pad quickly enough and/or because the superabsorbents are not able to "swell" sufficiently rapidly to absorb locally all of the liquid excreted. In other words, the liquid dispersion rate in the product is too low to be able to carry away large quantities of liquid from the wetting location with sufficient speed, by wetting location being meant the location in which the liquid is excreted onto the article. Under these conditions, the liquid will spread on the outer surface of the inner casing material and if the inner casing material should be inadvertently creased or folded when putting on the article, these creases or folds will be liable to function as a liquid flow channels and therewith result in leakage. It will be understood that the risk of leakage is greatest in the crotch area of the article, partly because the wetting location lies in this region of the article and partly because the absorbent pad is normally narrowest at this location. Furthermore, the crotch part of the absorbent pad normally always becomes deformed when the article is worn.

It is know from publication GB 2 124 907 that in the case of an article of the aforedescribed kind, the rate at which liquid is dispersed within an absorbent pad can be increased by incorporating in the pad a body of foamed-plastic material of very high liquid-permeability. The foamed-plastic body has a part which extends to the surface of the absorbent pad and which lies within the region of the wetting location when the article is worn. When liquid is excreted, the liquid flows through the part of the foam-plastic body located at the wetting location and when the absorbent pad is saturated locally, the liquid disperses quickly to dry parts of the pad through the agency of the foamed-plastic material. Publication US-A-4795453 discloses a disposable absorbent article having a body of a hose-like form and describes a similar article having the features stated in the introductory portion of Claim 1. Thus, the inclusion of such foamed-plastic bodies in articles of this kind will greatly increase the rate at which liquid is dispersed in the absorbent pad, although leakage can still nevertheless occur, for instance when the article concerned is an adult incontinence guard where the amount of liquid excreted, almost instantaneously, may be very large. The object of the present invention is to provide an absorbent disposable article whose absorbent pad has a high liquid dispersion rate and which is capable of absorbing large quantities of instantaneously excreted fluid.

This object is achieved in accordance with the invention with an absorbent disposable article of the afore-described kind having the characterising features of claim 1.

The hose-like body enables the liquid excreted to be carried away from the wetting location very quickly, therewith enabling large pacts of the absorbent pad material to be utilized rapidly. Furthermore, when a large quantity of liquid is excreted at one and the same time, the interior space of the hose-like body will function as a liquid storage space, therewith enhancing the ability of the article to take-up large quantities of liquid at any one time. Because the hose-like body is made from a shape-stable, liquid-permeable material, the hose-like body will constantly maintain a tubular shape, not withstanding the deformation to which the article is always subjected in the crotch part of the article when said article is placed in position on the wearer and during the subsequent use of said article, and consequently the internal space of the hose-like body can be utilized to take-up liquid and to ensure that excreted liquid will always be distributed in a suitable fashion. All of these factors combine to provide an absorbent, disposable article which, in accordance with the invention, is able to absorb large quantities of liquid with a high degree of reliability against leakage.

These and other features of the invention and advantages afforded thereby will be evident from the following detailed description of a preferred exemplifying embodiment of an inventive disposable absorbent article, said description being made with reference to the accompanying drawings, in which:
Figure 1 is a view from above of an inventive diaper in which the inner casing layer has been partially removed;
Figure 2 is a sectional view taken on the line II-II in Figure 1;
Figure 3 is a sectional view similar to that of Figure 2 but with the diaper placed in position on the wearer; and
Figures 4 and 5 illustrate examples of the manner in which a sheet-like body of shape-stable material can be deformed when putting-on a diaper.

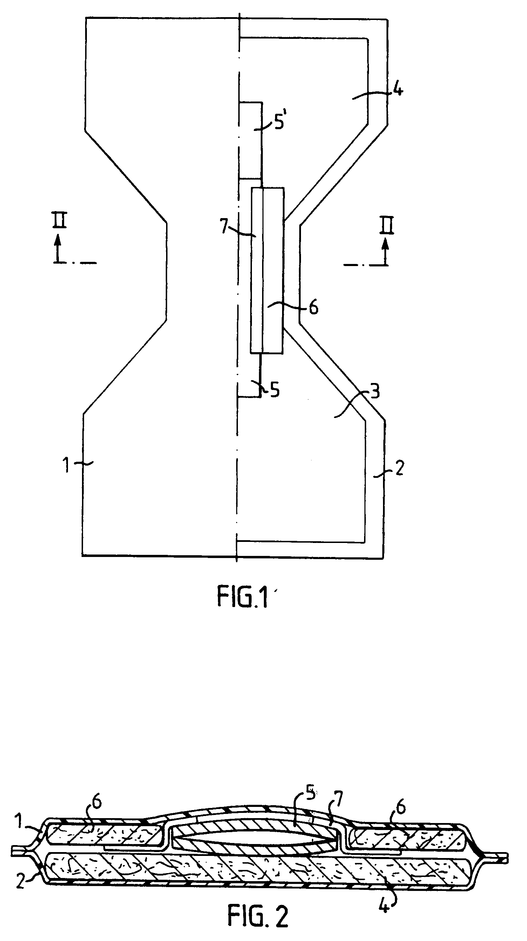

The diaper illustrated in Figures 1-3 includes, in a conventional manner, an inner liquid-permeable casing layer or sheet 1, which lies nearest the body of the wearer when the diaper is worn, and an outer liquid-impermeable casing layer or backing sheet 2. The inner and outer casing sheets are normally made of a nonwoven fibre material and polyethylene or polypropylene plastic material respectively. The inner and outer casing sheets enclose therebetween an absorbent pad 3 and are mutually joined together along those regions located externally of the absorbent pad.

The pad 3 is comprised of two mutually different layers. The outer layer comprises a highly-absorbent, hour-glass body 4. The inner layer comprises a central, hose-like body 5 made of a shape-stable material of very high liquid-permeability and two side-bodies 6 of rapidly-absorbing material. Examples of material from which the highly-absorbent body 4 can be made include so-called superabsorbents, compressed cellulose-fluff material or combinations of superabsorbents and cellulose-fluff materials or tissue. By rapidly-absorbing material is meant material which can absorb a limited quantity of liquid very rapidly. The rapidly-absorbing material may, for instance, comprise cellulose-fluff material which has large capillaries and which has not been compressed or compressed to only a slight degree. Such material, however, is not able to absorb the same amount of liquid per unit volume as the aforesaid material in the highly-absorbent body 4.

The shape-stable, liquid-permeable material in the hose-like body 5 preferably comprises fibre wadding in which the fibres are bound together in some suitable manner, for instance by thermo bonding, with the aid of a binding agent or are bound mechanically, so as to obtain a three-dimensional fibre structure. The fibre wadding may be comprised of natural fibres and other biodegradable fibres or of polyester, polypropylene or polyethylene fibres or mixtures of these fibres.

Although it is preferred to construct the hose-like body 5 from fibre wadding, it will be understood that foamed plastic, such as polyether foam, polyester foam or polyurethane foam can be used, as disclosed in the earlier mentioned publication GB 2 124 907.

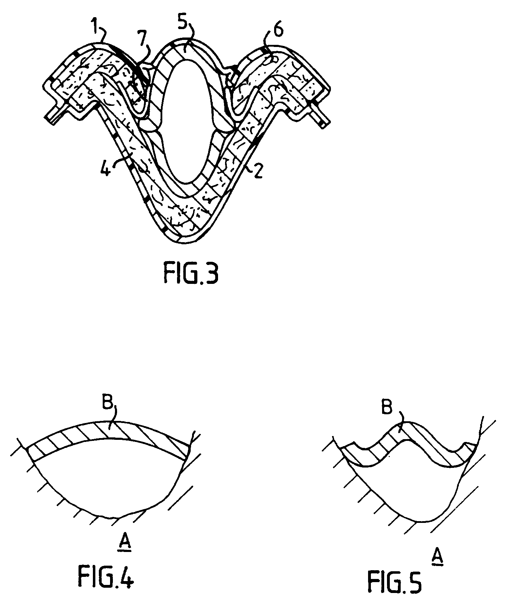

Figure 3 illustrates schematically how the diaper is deformed in the crotch part thereof subsequent to being placed on the wearer. As illustrated in this Figure, the hose-like body 5 will remain tubular, which means that the free space located inwardly of the walls of the hose-like body will increase. This is an important consequence of the configuration of said body, since this space functions as a storage space for excreted liquid, as explained in more detail hereinafter. Furthermore, the upper part of the hose-like body obtains an outwardly convex shape, which means that the uppermost part of the upper part of the body will lie against the body of the wearer within the region of the wetting location, particularly when the wearer is a female. This will ensure that excreted liquid will be distributed advantageously within the absorbent pad, with the aid of the hose-like body.

If a large quantity of liquid is excreted instantaneously onto the wetting location, the liquid will flow through the outwardly convex upper part of the hose-like-body, into the internal free space of said body, through the lower outwardly concave part of said body and into the highly-absorbent body 4 of hour-glass configuration. Because the absorption rate in the body 4 is not sufficiently high for large quantities of liquid to be absorbed rapidly, not all of the liquid excreted can flow into the body 4 and liquid will therefore fill the lowermost part of the hose-like body and also a part of the internal space thereof. The liquid-dispersing properties of the material in the hose-like body therewith cause liquid to be dispersed in the longitudinal direction of said body in the lowermost part thereof, therewith enabling liquid to be dispersed to drier parts of the body 4 of hour-glass configuration, and enable a larger part of the total absorption capacity of said body to be utilized. That part of the liquid excreted at one and the same time which is not immediately dispersed in the aforesaid manner will initially be stored in the interior space of the hose-like body and subsequently absorbed by the body 4 of hour-glass configuration at the same rate as the slower absorption rate of said body.

For the purpose of additionally guiding the flow of excreted liquid, two liquid barriers 7 are provided on respective sides of the hose-like body 5 between said body and the side-bodies 6. In the illustrated embodiment, these barriers simply comprise strips of liquid-impervious plastic, although the barriers may, of course, have other configurations within the scope of the inventive concept. For instance, the barriers may be provided by appropriate treatment of corresponding surfaces of the side-bodies 6. Neither need the barriers be totally liquid-impervious. The only essential criterion in this respect is that the liquid-permeability of the barriers is considerably less than the permeability of the hose-like body.

The function of the barriers 7 is to prevent liquid from flowing laterally from the hose-like body and into the side-bodies 6, so as to ensure that the excreted liquid will be absorbed primarily by the body 4 of hour-glass configuration. This reduced the risk of lateral leakage when the interior space of the hose-like body is filled to a high level. It is mentioned in this regard that the slope of the surface of the liquid held in the internal space of ±he hose-like body will, of course, depend on the attitude of the wearer's body, and hence the barriers also have a sealing function to reduce the risk of lateral leakage due to the slope of the liquid surface or to splashing of the liquid as a result of movement of the wearer. If these barriers are not provided, it is possible that the rapidly-absorbing side-bodies 6 will become saturated when coming into contact with the relatively large quantity of liquid stored within the hose-like body, resulting in lateral leakage. Furthermore, the side-bodies are provided with the intention of taking-up any liquid which may run on the surface of the inner casing sheet 1, so as to further reduce the risk of lateral leakage. This safety function would thus be jeapordized if the barriers 7 were not provided. It should be mentioned, however, that because of the presence of the inventive hose-like body 5 the risk of liquid escaping onto the inner casing sheet is very small, and consequently the provision of side bodies 6 and barriers 7 constitutes a preferred, but not absolutely necessary safety facility.

Figures 4 and 5 illustrate two examples of how a sheet-like body B comprising a form-stable, liquid-permeable material can be deformed when placing a diaper A on a wearer and while the diaper is being worn. The dispersion properties of the body B cannot be utilized in both of these cases, which greatly increases the risk of leakage. By forming the body 5 of highly liquid-permeable material to a hose-like configuration, it is ensured that the body will obtain a tubular configuration in the deformed state of a worn diaper irrespective of whether the compression forces to which the diaper is subjected when worn are symmetrical or not, which means that its lowermost part will always be in contact with liquid stored in the internal space of the hose-like body and that the liquid-dispersing properties of said body can thereby be utilized. Furthermore, because the uppermost part of the hose-like body has an outwardly convex shape, said uppermost part will abut the body of the wearer in the region of the wetting location, which ensures that the liquid excreted will be dispersed in the manner intended. Another contributory factor, in this regard, is that the aforesaid materials from which the hose-like body 5 can be made are all shape-stable in both a dry and wet state and have elastically restoring properties, i.e. when compressed they strive to return to their original form subsequent to the removal of the compressing force. When the diaper is worn by a member of the female sex, the uppermost part of the hose-like body will thus press against the genitals of the wearer, which ensures that the liquid excreted will always be deposited immediately into the hose-like body.

The hose-like body of the illustrated embodiment is manufactured by bonding two, flat elongated sheet-like bodies of bound fibre wadding along the edge margins thereof in some suitable manner, e.g, as by welding or gluing. Naturally, other methods of producing a hose-like body are conceivable, such as folding together an elongated, sheet-like material and securing the material along the two free edges thereof. The hose-like body is deformed to its tubular shape solely in the crotch part of the diaper, and the internal space of said body will therefore decrease successively and finally terminate completely in parts of the hose-like body located outwardly of the crotch-part. Consequently, the body 5 made of shape-stable, liquid-permeable material need not have the form of a hose throughout the whole of its length and a sheet-like part of the body 5 is indicated at 5′ in Figure 1. In accordance with one variant, the body 5 may, of course, initially have the tubular form illustrated in Figure 3 and the expression "hose-like" used in the Claims is not restricted to the hose-shape illustrated in Figure 2.

In summary, the invention provides a disposable absorbent article which is able to take-up a large quantity of instantaneously excreted liquid, with a good margin of safety against leakage. The hose-like body 5 will always be deformed in the manner desired when the article is placed on a wearer and will therefore positively achieve good dispersion and distribution of the excreted liquid throughout the absorbent material of the absorbent pad, while at the same time the internal space of the hose-like body will form a storage space for that excreted liquid which is not absorbed immediately by the hose-like body or by the absorbent pad material. Because the hose-like body is positioned centrally, the body will always lie within the region of the wetting location and the elastic restoring properties of the material from which the hose-like body is made will ensure that the inventive article will conform very readily to the body contours of the wearer, which together with the very high liquid-permeability of the hose-like body will ensure that excreted liquid is transported into the absorbent parts of the article in a desirable manner. The elastic restoring properties of the hose-like body also ensure that the side-parts of the article will be pressed sealingly against the thighs of the wearer when the article is worn.

It will be understood that such an article is particularly suitable for use as an incontinence guard for adult women. However, the invention can also be applied to advantage in conjunction with diapers for both large and small children. When the invention is applied in children's diapers, the ability to absorb large quantities of liquid excreted at one and the same time is not of a primary interest, and the liquid-dispersion and leakage-safety properties of the diaper are decisive for such an application.

It will be understood that the illustrated embodiment can be modified in many ways within the scope of the normal expertise of one skilled in this art. For instance, the hose-like body can be enveloped by an homogenous layer of absorbent material, instead of being surrounded by separate absorbent pads as in the illustrated embodiment. Furthermore, the shapes and dimensions of the parts incorportated in the absorbent pad can be varied. In particular, the upper layer of the absorbent pad may comprise a body of absorbent material having a shape which coincides with the body forming the bottom layer and provided with a central recess in which the hose-like body is placed, and the side-bodies and liquid barriers. can be excluded, as before mentioned. Furthermore, the casing material and the parts incorporated in the absorbent body can be joined together in different ways without departing from the concept of the invention. The scope of the invention is therefore only limited by the content of the following Claims.

## Claims

1. A disposable absorbent article, such as a diaper or an incontinence guard, which comprises an inner liquid-permeable casing sheet (1), which is intended to lie nearest the body of the wearer when the article is worn, an outer liquid-impermeable casing sheet (2), and an absorbent pad (3) enclosed between said two casing sheets, the absorbent pad, at least within the region thereof which forms the crotch part when the article is worn, includes a body (5) of a hose-like form comprising a shape-stable material of very high liquid-permeability, the body being centrally positioned and extending in the longitudinal direction of the article bordering on the inner casing sheet (1) and being surrounded by absorbent material (4) on at least the side thereof remote from the inner casing sheet, **characterized in** that the part of the hose-like body (5) intended to face the wearer during use of the article has an outwardly convex shape intended to abut the body of the wearer in the region of the wetting location, when the article is worn.

2. An article according to Claim 1, **characterized** in that the hose-like body (5) is made of fibre wadding in which the fibres are bound together to form a three-dimensional fibre structure.

3. An article according to Claim 2, **characterized** in that the fibre wadding is comprised of polyester fibres, polypropylene fibres or polyethylene fibres or mixtures thereof.

4. An article according to Claim 1, **characterized** in that the hose-like body (5) is made of a foamed-plastic material.

5. An article according to Claim 4, **characterized** in that the foamed-plastic material is a polyether plastic, a polyester plastic or a polyurethane plastic.

6. An article according to any one of the preceding Claims, **characterized** in that the absorbent pad (3) is comprised of a body (4) of highly-absorbent material and of hour-glass configuration which lies nearest the outer casing sheet (2), and two side-bodies (6) of rapidly-absorbing material located inwardly of said body and on opposite sides of the hose-like body (5).

7. An article according to any one of the preceding Claims, **characterized** in that two liquid barriers (7) are provided on respective sides of the hose-like body (5) and border on said body.

8. An article according to Claim 7, **characterized** in that the barriers (7) consist of strips of liquid-impervious plastic material.

## Patentansprüche

1. Absorbierender Wegwerfartikel, wie beispielsweise eine Windel oder ein Inkontinenzschutz, der eine innere flüssigkeitsdurchlässige Decklage (1), die dazu bestimmt ist, nächst der Trägerperson zu liegen, wenn der Artikel getragen wird, eine äußere flüssigkeitsundurchlässige Decklage (2) und ein zwischen den zwei Lagen eingeschlossenes absorbierendes Kissen aufweist, wobei das absorbierende Kissen zumindest in demjenigen Bereich, der den Schrittabschnitt bildet, wenn der Artikel getragen wird, einen Körper (5) mit schlauchartiger Form enthält, der ein formstabiles Material mit sehr hoher Flüssigkeitsdurchlässigkeit umfasst, wobei der Körper mittig positioniert ist und sich in Längsrichtung des Artikels, angrenzend an die innere Decklage (1) erstreckt und von dem absorbierenden Material (4) an zumindest derjenigen Seite umgeben ist, die von der inneren Decklage abgewandt ist, **dadurch gekennzeichnet, dass** derjenige Teil des schlauchartigen Körpers (5), der während der Benutzung des Artikels dazu bestimmt ist, der Trägerperson zugewandt zu sein, eine auswärts konvexe Form hat, die, wenn der Artikel getragen wird, dazu bestimmt ist, im Einnässungsbereich an den Körper der Trägerperson anzuliegen.

2. Absorbierender Wegwerfartikel nach Anspruch 1, **dadurch gekennzeichnet, dass** der schlauchartige Körper (5) aus einer Faserwattierung hergestellt ist, bei der die Fasern miteinander verbunden sind, um einen dreidimensionalen Faseraufbau zu bilden.

3. Absorbierender Wegwerfartikel nach Anspruch 2, **dadurch gekennzeichnet, dass** die Faserwattierung aus Polyesterfasern, Polypropylenfasern oder Polyethylenfasern oder Mischungen davon besteht.

4. Absorbierender Wegwerfartikel nach Anspruch 1, **dadurch gekennzeichnet, dass** der schlauchartige Körper (5) aus einem geschäumten Kunststoffmaterial besteht.

5. Absorbierender Wegwerfartikel nach Anspruch 4, **dadurch gekennzeichnet, dass** das geschäumte Kunststoffmaterial ein Polyether-, Polyester- oder ein Polyurethan-Kunststoff ist.

6. Absorbierender Wegwerfartikel nach einem der voranstehenden Ansprüche, **dadurch gekennzeichnet, dass** das absorbierende Kissen (3) aus einem nächst der äußeren Decklage (2) liegenden und eine Sanduhrform aufweisenden Körper (4) aus hochabsorbierendem Material und zwei Seitenkörper (6) aus schnell absorbierendem Material besteht, die innerhalb des Körpers und an gegenüberliegenden Seiten des schlauchartigen Körpers (5) liegen.

7. Absorbierender Wegwerfartikel nach einem der voranstehenden Ansprüche, **dadurch gekennzeichnet, dass** an den jeweiligen Seiten des schlauchartigen Körpers (5) zwei Flüssigkeitsbarrieren (7) angeordnet sind und an den Körper angrenzen.

8. Absorbierender Wegwerfartikel nach Anspruch 7, **dadurch gekennzeichnet, dass** die Flüssigkeitsbarrieren (7) aus Streifen aus flüssigkeitsundurchlässigem Kunststoffmaterial bestehen.

## Revendications

1. Article absorbant jetable, comme une couche ou une protection pour incontinents, qui consiste en une feuille (1) formant enveloppe interne perméable au liquide qui est destinée à se trouver à proximité du corps de l'utilisateur quand l'article est porté, une feuille (2) formant enveloppe externe imperméable au liquide, et un coussin (3) absorbant enfermé entre lesdites deux feuilles formant enveloppe, le coussin absorbant, au moins dans sa région qui forme la partie de l'entre-jambes quand l'article est porté, comporte un corps (5) en forme de tuyau composé d'un matériau de forme stable ayant une très forte perméabilité au liquide, le corps étant positionné de façon centrale et s'étendant dans le sens longitudinal de l'article, jouxtant la feuille (1) formant enveloppe interne, et étant entouré d'un matériau absorbant (4) sur au moins son côté qui est éloigné de la feuille (1) formant enveloppe interne, caractérisé en ce que la partie du corps (5) en forme de tuyau, destinée à se trouver face à l'utilisateur pendant l'utilisation de l'article a une forme convexe vers l'extérieur destinée à venir toucher le corps de l'utilisateur dans la région de l'endroit de mouillure quand l'article est porté.

2. Article suivant la revendication 1, caractérisé en ce que le corps (5) semblable à un tuyau est fait d'un capitonnage de fibres dans lequel les fibres sont liées ensemble pour former une structure de fibres tridimensionnelle.

3. Article suivant la revendication 2, caractérisé en ce que le capitonnage de fibres est constitué de fibres de polyester, fibres de polypropylène ou fibres de polyéthylène, ou de mélanges de celles-ci.

4. Article suivant la revendication 1, caractérisé en ce que le corps (5) semblable à un tuyau est fait d'un matériau en plastique expansé.

5. Article suivant la revendication 5, caractérisé en ce que le matériau en plastique expansé est un plastique polyéther, un plastique polyester, ou un plastique polyuréthane.

6. Article suivant l'une quelconque des précédentes revendications, caractérisé en ce que le coussin absorbant (3) est constitué d'un corps (4) en matériau fortement absorbant, ayant une configuration de verre de montre, qui est placé le plus près de la feuille (2) qui forme enveloppe extérieure, et de deux corps latéraux (6) en matériau à absorption rapide, situés vers l'intérieur dudit corps et sur les côtés opposés du corps (5) en forme de tuyau.

7. Article suivant l'une quelconque des précédentes revendications, caractérisé en ce que deux barrières (7) à liquide sont placées sur les côtés respectifs du corps (5) en forme de tuyau et touchent ledit corps.

8. Article suivant la revendication 7, caractérisé en ce que les barrières (7) consistent en des bandes de matériau plastique imperméable au liquide.
